# FASCICULE DE BREVET EUROPEEN

(11) **EP 0 605 319 B1**
(45) Date de publication et mention de la délivrance du brevet: **02.09.1998**
(21) Numéro de dépôt: 93403187.3
(22) Date de dépôt: 27.12.1993
(51) Int. Cl.: C12Q 1/04, C12Q 1/24

(54) **Méthode de diagnostic rapide de bactéries aérobies présentes dans un milieu biologique**
Diagnostisches Schnellverfahren für aerobe Bakterien in einem biologischen Medium
Rapid diagnostic method for aerobic bacteria present in a biological medium

(30) Priorité: 29.12.1992 FR 9215854
(43) Date de publication de la demande: 06.07.1994
(73) Titulaire: ASSISTANCE PUBLIQUE, 75100 Paris (FR)
(72) Inventeur: Nicolas, Marie-Hélène, F-75004 Paris (FR); Bru, Fabrice, F-92150 Suresnes (FR)
(74) Mandataire: Le Guen, Gérard

(56) Documents cités:
- JOURNAL OF CLINICAL MICROBIOLOGY vol. 3 , Mars 1976 pages 258 - 263 G.L.DORN 'Blood Culture Technique Based on Centrifugation: Clinical Evaluation'
- JOURNAL OF CLINICAL MICROBIOLOGY vol. 16, no. 6 , Décembre 1982 pages 1052 - 1056 C.J.SHANHOLZER ET AL. 'Concentrated Gram Stain Smears Prepared with a Cytospin Centrifuge'

## Description

La présente invention concerne une méthode de diagnostic rapide de bactéries aérobies présentes dans un milieu biologique et notamment dans le sang.

En dépit des antibiotiques, les septicémies restent une cause majeure de mortalité. Ces dernières années, on a proposé de traiter les septicémies et les chocs septiques à Gram négatif par des anticorps monoclonaux anti-endotoxine. Toutefois, ces traitements coûtent cher et il est donc logique de ne les prescrire qu'aux malades qui sont l'objet d'une septicémie à Gram négatif. Faire la preuve de l'existence de bactéries à Gram négatif dans le sang ne passe que par deux voies possibles : doser l'endotoxine ou mettre en évidence les bactéries à Gram négatif.

Il s'avère toutefois que les méthodes de diagnostic utilisées pour cette dernière voie donnent des réponses beaucoup trop tardives compte tenu de la nécessité qu'il y a à administrer rapidement les anticorps anti-endotoxine pour qu'ils soient efficaces. Ainsi, les méthodes classiques mettant en oeuvre des cultures ne permettent pas un diagnostic en moins de 12 heures.

On a notamment décrit des méthodes de diagnostic utilisant des opérations de lyse et de centrifugation du prélèvement sanguin avant la mise en culture dudit prélèvement (voir par exemple G. Dorn et al., J. Clinical Microbiology, 1976, 3 : 258-263 et N. Henry et al., J. Clinical Microbiology, 1983, 17 : 864-869). Toutefois le diagnostic passe toujours pas une culture avec un temps d'incubation minimal de 18 heures.

On a également décrit des méthodes comprenant une détection de la croissance bactérienne par radiométrie. La détection ne peut toutefois être effectuée que dans un minimum de 12 heures.

La présente invention vise à fournir une méthode de diagnostic rapide, c'est-à-dire au maximum en 5 heures, des bactéries aérobies présentes dans un milieu biologique et notamment dans le sang, même à des concentrations très faibles (0,1 à 10 UFC/ml).

La présente invention a ainsi pour objet une méthode de diagnostic rapide de bactéries aérobies présentes dans un milieu biologique comprenant successivement :
a) une culture rapide (maximum 4 heures) en aérobiose d'un échantillon du milieu biologique sous agitation,
b) une centrifugation rapide pour récupérer les bactéries dans le culot de centrifugation,
c) la projection par centrifugation du culot précédemment obtenu sur un support
d) la révélation des bactéries sur ledit support.

La présente invention a plus particulièrement pour objet une méthode de diagnostic de bactéries aérobies présentes dans le sang comprenant successivement:
a) une culture en aérobiose d'un échantillon de sang sous agitation, pendant de 2 à 4 heures,
b) une séparation des hématies par centrifugation lente et une récupération du surnageant,
c) une lyse des globules blancs dans le surnageant,
d) une centrifugation rapide du milieu ainsi obtenu pour récupérer les bactéries dans le culot de centrifugation,
e) la projection par centrifugation du culot précédemment obtenu sur un support,
f) la révélation des bactéries sur ledit support.

La première étape de la méthode selon l'invention est une culture d'un échantillon, par exemple de 8-10 ml de sang sous agitation forte, de préférence sous agitation très forte en présence d'oxygène, par exemple en présence d'air. L'agitation peut être effectuée à des vitesses de l'ordre de 100 à 400 tours par minutes, par exemple 250 tours par minute. Cette étape va à l'encontre de tout ce qui était recommandé jusqu'ici. En effet, en général l'agitation est inexistante, ou faible et de très courte durée afin de permettre le dépôt des hématies. Dans la présente invention, la culture sous agitation est effectuée généralement pendant une durée de 2 à 4 heures, cette durée pouvant être d'autant plus courte que la concentration en bactéries est plus élevée.

En l'absence d'une telle agitation le seuil des bactéries qui peuvent être détectées est beaucoup plus élevé et le délai pour l'obtention des résultats est double. Ainsi, en l'absence d'agitation et en utilisant les mêmes étapes successives, il n'est possible de mettre en évidence qu'au minimum des concentrations de 10² UFC de Escherichia coli/ml de sang et qu'après 8 heures de culture, alors qu'en effectuant la culture sous agitation pendant 4 heures, on peut mettre en évidence des concentrations de *Escherichia coli* aussi faibles que 0,1 UFC/ml.

L'étape de séparation des hématies par centrifugation lente peut être effectuée notamment à 200-300 g pendant une durée de l'ordre de 15 minutes.

L'étape de lyse des globules blancs peut être effectuée comme décrit par N. Henry (cité ci-dessus) en utilisant le produit contenu dans les tubes Isolator ou tout autre produit permettant la lyse, tel que la saponine (concentration finale de 0,2%). A cet effet le produit permettant la lyse est ajouté directement au surnageant et le tout est mélangé intimement, par exemple à l'aide d'un vortex pendant 1 minute.

L'étape de centrifugation rapide du milieu ayant subi la lyse est réalisée pour concentrer les bactéries. Cette centrifugation peut être effectuée à 4000-5000 g pendant 10 minutes.

L'étape de projection par centrifugation du culot précédemment obtenu sur un support peut être effectuée notamment en utilisant un appareil appelé Cytospin (voir en particulier C. Shanholtzer et al., J. Clinical Microbiology, 16, 6, 1052, 1982) ou tout autre principe de centrifugation. Cet appareil permet de projeter et concentrer les bactéries contenues dans le culot sur une faible surface d'une lame de verre c'est-à-dire sur un cercle d'environ 4 mm de diamètre permettant ainsi d'effectuer une révélation des bactéries sur le support. Cette révélation peut être effectuée en général par coloration de Gram.

La méthode selon l'invention a été mise au point en utilisant 3 souches de bactéries aérobie rencontrées dans les infections nosocomiales : *Escherichia coli* , *Pseudo-monas aeruginosa* et *Acinetobacter baumanii.*

Ces bactéries ont été cultivées sur gélose pendant 18 heures en aérobiose. Des colonies ont été prélevées et diluées dans 2 ml d'eau distillée stérile et l'on a obtenu des concentrations de bactéries de 5.10⁶ à 5.10⁸ UFC/ml. Des échantillons de sang de 10 ml ont été inoculés avec 0,1 ml de dilutions 10⁻⁵ et 10⁻⁶ pour chaque espèce, la densité des bactéries dans les échantillons étant de 0,05 à 5 UFC/ml.

Les échantillons de sang (10 ml) ont été transférés dans des flacons d'hémoculture contenant 20 ml de bouillon de culture (Hemoline performance Biomérieux S.A.) avec 0,025 % de polyanetholsulfonate de sodium et mis à incuber sous agitation (250 tours par minute) (Rotatest, Bioblock) pendant 4 heures à 37° C.

Ensuite chaque culture a été soumise à une centrifugation à 250 g pendant 15 minutes à 20° C. Le surnageant a été prélevé et pour effectuer la lyse des globules blancs le composé Isolator (Dupont) ou de la saponine à la concentration finale de 0,2 % a été ajouté. Le mélange a été agité au Vortex pendant 1 minute puis centrifugé à 4500 g pendant 10 minutes. Après élimination du surnageant, le culot a été soumis dans sa totalité à une centrifugation à 192 g pendant 10 minutes dans une cytocentrifugeuse (Cytospin 2, Shandon) pour obtenir sur une lame de verre un frottis de 4 mm de diamètre. La coloration de Gram a été effectuée sur cet échantillon et examinée au microscope à immersion.

On a pu ainsi détecter la présence d'*E. coli* à la concentration de 0,1 UFC/ml dans un échantillon de sang, de *P. aeruginosa* à la concentration de 0,3 UFC/ml et de *A. baumanii* à la concentration de 1,5 UFC/ml.

En outre, en l'absence d'antibiotique dans les échantillons de sang, la méthode conduit à mettre en évidence la morphologie des bactéries permettant ainsi une orientation diagnostique quant au type d'espèce. Ainsi, *E. coli* est reconnaissable sous forme de batonnet épais, *P. aeruginosa* sous forme de batonnet fin et allongé, *A. baumanii* sous forme de coccobacilles. Ceci est un avantage supplémentaire de la méthode car cela permet au clinicien de choisir de façon plus précise le traitement antibiotique.

Enfin, sur le culot d'hématies séparées grâce à la centrifugation lente il est possible d'effectuer un antibiogramme le même jour, d'où un gain de temps appréciable.

Il est à noter que la méthode s'applique non seulement au diagnostic de *Pseudomonas aeruginosa, Acinetobacter* et *Escherichia coli* mais également à d'autres entérobactéries telle que *Klebsiella pneumoniae,* d'autres bacilles à Gram négatif dont *Haemophilus influenzae* qui est un germe exigeant des facteurs de croissance, des cocci à Gram négatif tel que *Neisseiria meningitidis* et enfin des cocci à Gram positif tels que *Staphylococcus aureus, Enterococcus,* streptocoques β-hémolytiques.

L'application de la méthode à du sang prélevé chez 39 malades, considérés cliniquement septicémiques ou en choc septique a permis de calculer la sensibilité et la spécificité de la méthode en référence aux hémocultures classiques (Flacon hémoline anaérobie de BioMérieux, Marcy l'Etoile) et à l'Isolator (Merck). Pour ces deux méthodes, la culture directe ou le repiquage ont été faits sur gélose chocolat incubée en atmosphère de CO₂ (5 %) et sur gélose au sang incubée en aérobiose.

Le tableau I donne une liste des espèces bactériennes qui ont été isolées, la concentration sanguine initiale appréciée par la culture de l'Isolator et les résultats morphologique et de coloration de gram données par la méthode de diagnostic rapide selon l'invention.

**TABLEAU 1**

| **Cas** | **Espèce** | **Concentration sanguine UFC/ml** | **Morphologie et Gram apprécié après diagnostic rapide** |
|---|---|---|---|
| 7 | *P. aeruginosa* | 0,1 | Bacille à Gram négatif |
| 8 | *S. aureus* | 0,5 | Cocci à Gram positif en amas |
| 9 | *P. aeruginosa* | 0,8 | Bacille à Gram négatif |
| 22 | entérocoque | 2,8 | Cocci à Gram positif en chaînette |
| 32 | *E. coli* | 32 | Bacilles à Gram négatif |
| 33 | *E. coli* | 2,5 | Bacilles à Gram négatif |
| 35 | *Serratia Spp* | 7,1 | Bacilles à Gram négatif |
| 38 | *Bacteroïdes fragilis* | - | Bacille à Gram négatif |

Il est à noter que pour le cas 38 les hémocultures classiques en anérobiose mais pas l'Isolator ont permis d'isoler une souche de *Bacteroïdes fragilis* (bactérie anaérobie). Par la méthode de diagnostic rapide, il avait été répondu présence de bacilles à Gram négatif. Par cette dernière méthode la culture qui est faite en aérobiose n'a normalement pas pu permettre la multiplication de la souche. Ceci sous-entendrait que la concentration initiale de *Bacteroïdes fragilis* dans le sang du malade était importante et suffisante pour que la simple étape de concentration des bactéries ait permis de les mettre en évidence par la coloration de Gram.

De plus, pour 5 cas dont les cultures du sang sont demeurées négatives par les méthodes classiques, la méthode de diagnostic rapide a été positive. Dans 4 cas, il a été répondu présence de bacilles à Gram négatif et pour le dernier, présence de rares cocci à Gram positif en amas. Pour un des cas à bacilles à Gram négatif, il est à noter que les bacilles étaient très polymorphes et en très grande quantité alors que dans les autres cas la quantité de bacilles était faible comme dans le cas des cocci à Gram positif.

La méthode rapide a aussi permis de mettre en évidence la présence dans le sang des malades de bacilles anormaux en terme de morphologie (bacilles déformés non longiformes) et en terme de coloration de Gram (granulation, coloration inhomogène, Gram positif et Gram négatif). Ces bacilles "anormaux" ne sont d'une part pas cultivables et d'autre part aussi présents dans le sang des sujets sains. A la différence des sujets sains la quantité de ces bacilles "anormaux" est parfois plus importante chez les sujets présumés cliniquement septicémiques et/ou en choc septique.

L'ensemble de ces derniers résultats montre que la méthode de diagnostic rapide, grâce à son étape de concentration des bactéries par la centrifugation est non seulement capable de détecter des bactéries viables mais également des bactéries non viables.

La méthode selon l'invention avec culture agitée et rapide a pu être étendue à d'autres types de prélèvements que le sang. En effet, cette méthode est également performante pour mettre en évidence la présence de bactéries dans le liquide céphalo-rachidien (LCR). Les premières expériences faites *in vitro* montrent qu'une culture de 1 ml de LCR dans 10 ml de bouillon de culture (hémoline) est détectée positive par la méthode rapide, lorsque le LCR a été contaminé *in vitro* par 10 UFC/ml de *S. aureus, S. epidermidis, E. coli*, *K. pneumoniae*, *P. aeruginosa, Listeria monocytogenes* ou streptocoque.

Toutefois, pour appliquer cette méthode au LCR, quelques modifications ont été apportées :
- il n'y a plus de centrifugation lente, car il n'y a pas beaucoup de globules rouges dans le LCR,
- la lyse n'a pas été effectuée car la lyse des leucocytes en grande quantité dans le LCR augmente le bruit de fond lors de la coloration de Gram.

## Revendications

1. Méthode de diagnostic rapide de bactéries aérobies présentes dans un milieu biologique comprenant successivement :
a) une culture rapide en aérobiose d'un échantillon du milieu biologique sous agitation, pendant de 2 à 4 heures,
b) une centrifugation rapide pour récupérer les bactéries dans le culot de centrifugation,
c) la projection par centrifugation du culot précédemment obtenu sur un support,
d) la révélation des bactéries sur ledit support.

2. Méthode de diagnostic rapide de bactéries aérobies présentes dans le sang, comprenant successivement :
a) une culture en aérobiose d'un échantillon de sang sous agitation, pendant de 2 à 4 heures,
b) une séparation des hématies par centrifugation lente et une récupération du surnageant,
c) une lyse des globules blancs dans le surnageant,
d) une centrifugation rapide du milieu ainsi obtenu pour récupérer les bactéries dans le culot de centrifugation,
e) la projection par centrifugation du culot précédemment obtenu sur un support,
f) la révélation des bactéries sur ledit support.

3. Méthode selon la revendication 1 ou la revendication 2, dans laquelle la culture sous agitation est effectuée pendant de 2 à 4 heures 250 tours par minute.

4. Méthode selon l'une quelconque des revendications 1 à 3, dans laquelle la centrifugation lente est effectuée à 200-300 g.

5. Méthode selon l'une quelconque des revendications précédentes, dans laquelle la centrifugation rapide est effectuée à 4000-5000 g.

6. Méthode selon l'une quelconque des revendications précédentes dans laquelle la révélation est une coloration de Gram.

## Patentansprüche

1. Verfahren zur schnellen Diagnose von aeroben Bakterien, die in einen biologischen Milieu vorhanden sind, wobei das Verfahren nacheinander enthält:
a) eine schnelle Züchtung in Aerobiose einer Probe des biologischen Milieus unter Umrühren während 2 bis 4 Stunden,
b) eine schnelle Zentrifugierung, um die Bakterien im Zentrifugenrückstand zurückzugewinnen,
c) das Schleudern des vorher erhaltenen Rückstands auf einen Träger mittels Zentrifugierung,
d) die Entwicklung der Bakterien auf dem Träger.

2. Verfahren zur schnellen Diagnose von im Blut vorhandenen aeroben Bakterien, wobei das Verfahren nacheinander enthält:
a) eine Züchtung in Aerobiose einer Blutprobe unter Umrühren während 2 bis 4 Stunden,
b) eine Trennung der Erythrozyten durch langsame Zentrifugierung und eine Rückgewinnung des Serums,
c) eine Auflösung der Leukozyten im Serum,
d) eine schnelle Zentrifugierung des so erhaltenen Milieus, um die Bakterien im Zentrifugenrückstand zurückzugewinnen,
e) das Schleudern des obenerhaltenen Rückstands auf einen Träger mittels Zentrifugierung,
f) die Entwicklung der Bakterien auf dem Träger.

3. Verfahren nach Anspruch 1 oder Anspruch 2, in dem die Züchtung unter Umrühren während 2 bis 4 Stunden bei 250 Umdrehungen pro Minute ausgeführt wird.

4. Verfahren nach irgendeinem der Ansprüche 1 bis 3, bei dem die langsame Zentrifugierung mit 200-300 g erfolgt.

5. Verfahren nach irgendeinem der vorangehenden Ansprüche, bei dem die schnelle Zentrifugierung mit 4000-5000 g erfolgt.

6. Verfahren nach irgendeinem der vorangehenden Ansprüche, bei dem die Entwicklung eine Gram-Färbung ist.

## Claims

1. Method for the rapid diagnosis of aerobic bacteria present in a biological medium comprising successively:
a) rapid culturing (maximum 4 hours) in aerobiosis of a sample of the biological medium, accompanied by stirring,
b) rapid centrifuging to recover the bacteria in the centrifuging mass,
c) spraying by centrifuging of the previously obtained mass onto a support,
d) revealing the bacteria on said support.

2. Method for the diagnosis of aerobic bacteria present in blood comprising successively:
a) culturing in aerobiosis of a blood sample, accompanied by stirring, for 2 to 4 hours,
b) separation of erythrocytes by slow centrifuging and recovery of the supernatant product,
c) lysis of the white corpuscles in the supernatant product,
d) rapid centrifuging of the thus obtained medium to recover the bacteria in the centrifuging mass,
e) spraying by centrifuging the previously obtained mass onto a support,
f) revealing bacteria on said support.

3. Method according to claim 1 or 2, wherein culturing accompanied by stirring is performed for 2 to 4 hours at 250 r.p.m.

4. Method according to any one of the claims 1 to 3, wherein slow centrifuging is performed at 200 to 300 g.

5. Method according to any one of the preceding claims, wherein fast centrifuging is performed at 4000 to 5000 g.

6. Method according to any one of the preceding claims, wherein Gram staining revealing is used.
